# EUROPEAN PATENT APPLICATION

(11) **EP 3 591 665 A1**
(43) Date of publication of application: **08.01.2020**
(21) Application number: 18182007.7
(22) Date of filing: 05.07.2018
(51) Int. Cl.: G16H 50/30, A61B 5/16, A61B 3/113

(54) **METHOD FOR EVALUATING A RISK OF NEURODEVELOPMENTAL DISORDER WITH A CHILD**

(71) Applicant: Université Libre de Bruxelles, 1050 Bruxelles (BE)
(72) Inventor: Kissine, Mikhail, 1190 Forest (BE); Deliens, Gaétane, 1800 Vilvoorde (BE)
(74) Representative: ABYOO

(57) **Abstract**

A method is provided in order to evaluate a risk of child having a neurodevelopmental disorder. Firstly, a first and a second information are shown to the child at two different places on a display screen (5). Then the first and second information are removed from the display screen (5) for a while and meanwhile movements of the eye of the child are tracked and recorded. Next, a third information is displayed on the display screen (5), at a location where the second information was previously displayed. This sequence is repeated a number of times. Next, a score for the child is established in function of the movements of his eye(s) as recorded while the first and second information were removed from the display screen (5). This score gives an indication of a risk of neurodevelopmental disorder with the child.

## Description

### Field of the invention

The invention relates to methods and devices for evaluating a risk of a neurodevelopmental disorder, more particularly of an Autism Spectrum Disorder (ASD), with children.

### Description of prior art

International patent publication WO 2015/057321 discloses a method which, though fundamentally different, is in a field which is close to the field of the invention.

According to this known method, first eye movement data of a child is collected in the course of a first session during which a first visual stimulus is shown to the child and second eye movement data of the same child is collected in the course of a second session during which a second visual stimulus is shown to the child. The first and second eye movement data are then compared with each other to identify a change in visual fixation by the child on the two visual stimuli. A decline in visual fixation is described as being a marker of a developmental, cognitive, social or mental disability or ability of the child. This method, which is thus based on an evolutionary curve of fixations, can lead to significant differences at the level of a group performance, but it is not sensitive enough to allow the establishment of individual thresholds.

International patent publication WO2014/164858 discloses a method which, though fundamentally different, is also in a field which is close to the field of the invention. According to this known method, visual information is shown to a patient and fixations of the patient with respect to this information is analyzed and compared in order to calibrate the system and later to verify its accuracy when in operation.

Though such methods may provide interesting results for groups of persons, they lack accuracy when applied to individuals.

### Summary of the invention

It is an object of the invention to provide a method and device which deliver more accurate results for individuals, more particularly results with less false positive results and/or less false negative results.

The invention is defined by the independent claims. The dependent claims define advantageous embodiments.

According to the invention, there is provided a method for evaluating a risk of developmental disorder with a child, the method comprising the steps of :
- providing a machine comprising a display screen, an eye-tracking device, a controller configured for displaying information on the display screen and for reading and recording eye position data from the eye-tracking device;
- having the child passing a test in the course of a first time period, the test comprising, in sequence:
   a) having the child looking at the display screen (5) and directing the eye-tracking device (10) towards at least one eye of said child,
   b) displaying first information and second information at respectively two distinct locations on the display screen (5),
   c) removing the first information and the second information from the display screen (5),
   d) while the first information and the second information are removed from the display screen (5), reading and recording eye position data from the eye-tracking device (10), thereby obtaining a recorded movement of the at least one eye of the child,
   e) displaying third information on the display screen (5), at or close to the previous location of the second information, and optionally displaying again the second information on the display screen (5), at its previous location,
   f) removing the third information from the display screen (5),
   g) repeating steps b) to f) a finite number of times,
   h) calculating a score in function of the plurality of recorded movements of the at least one eye of the child obtained in the steps d).

Preferably, based on the recorded movement of the at least one eye of the child during each step d), the controller determines if the child made a fixation on the location on the display screen which was at least partially there where the second information was displayed during respectively each step b), and, in step h), the score is calculated in function of a number of said fixations or on an evolution of a number of said fixations over time (i.e. over the successive executions of steps b) to f)).
Alternatively, in step h), the score is calculated in function of a comparison of data from the plurality of recorded movements of the at least one eye of the child obtained in the steps d) with pre-recorded reference data.

A method according to the invention is more reliable than the known methods because the known methods only take into account regions of an image or video which are preferentially observed by the child (such as adult face versus an object), whereas a child may be attracted to a particular region of an image or video for various reasons. With known methods, inter-group discrimination will therefore be limited. By adding an anticipation paradigm into the process (the display of the third information overlapping the second information), we can come across this stumbling block.

It is to be stressed that the score resulting from the method according to the invention is in only indicative of a risk of a neurodevelopmental disorder with the child to whom it is applied and that it does neither provide a diagnostic per se of such a disorder nor does it presuppose in any way of a later diagnostic of such disorder.

Preferably, the neurodevelopmental disorder is an Autism Spectrum Disorder.

Preferably, the first time period is a within a period during which the child has an age comprised between 7 and 20 months, preferably between 9 and 18 months. More preferably, the first time period is a within a period during which the child has an age comprised between 9 and 11 months, or between 12 and 14 months.

The invention also provides an apparatus for evaluating a risk of neurodevelopmental disorder with a child, said apparatus comprising a display screen, an eye-tracking device and a controller, said controller being configured for, in the course of a first time period:
a) displaying first information and second information at respectively two distinct locations on the display screen (5),
b) removing the first information and the second information from the display screen (5),
c) while the first information and the second information are removed from the display screen (5), reading and recording eye position data from the eye-tracking device (10), thereby obtaining a recorded movement of the at least one eye of the child,
d) displaying third information on the display screen (5), at or close to the previous location of the second information, and optionally displaying again the second information on the display screen (5), at its previous location,
e) removing the third information from the display screen (5),
f) repeating steps a) to e) a finite number of times,
g) calculating a score in function of the plurality of recorded movements of the at least one eye of the child obtained in the steps c).

### Short description of the drawings

These and further aspects of the invention will be explained in greater detail by way of examples and with reference to the accompanying drawings in which:
- Fig.1: shows a flowchart of an exemplary method according to the invention;
- Fig.2: shows an example of a series of display screens that are shown to a child during execution of the method of Fig.1;
- Fig.3: schematically shows an apparatus according to the invention.

The drawings of the figures are neither drawn to scale nor proportioned. Generally, similar or identical components are denoted by the same reference numerals in the figures.

### Detailed description of embodiments of the invention

According to the invention, there is provided a method for evaluating a risk of neurodevelopmental disorder with a child.
The method first comprises the step of providing a machine comprising a display screen (5), an eye-tracking device (10), a controller (15) configured for displaying information on the display screen (5) and for reading and recording eye position data from the eye-tracking device (10).
The machine may for example be a portable personal computer or a tablet computer to which an eye tracking device is connected. Various commercially available eye tracking devices may be used, such as for example an Eye Tracker 4C model from Tobii Technology or a RED-m from SensoMotoric Instruments or a RK-464 model from ISCAN Inc. or an TM5 Mini from EyeTech. These eye-tracking devices come with appropriate control software which, once installed and run, enable the controller (15) (which is generally the computer itself) to read and record eye position data from the eye-tracking device (10). The machine may for example also be a portable personal computer or a tablet computer with a built-in eye tracking device and corresponding control software. The display screen (5) may be an individual video screen or a video screen integrated into another device such as a personal computer or a tablet computer or a video projector or any other electronic means for displaying images.
The controller (15) may be a dedicated controller (15) or a personal computer or a tablet computer or any other means having the capability to display information on the display screen (5), to read and record eye position data from the eye-tracking device (10), to perform operations on these data, to read data from a data storage medium and to save results on a data storage medium.

Once the machine is set-up, a child to be examined passes a test in the course of a first time period, the test comprising, in sequence:
a) having the child looking at the display screen (5) and directing the eye-tracking device (10) towards at least one eye of said child,
b) displaying first information and second information at respectively two distinct locations on the display screen (5),
c) removing the first information and the second information from the display screen (5),
d) while the first information and the second information are removed from the display screen (5), reading and recording eye position data from the eye-tracking device (10), thereby obtaining a recorded movement of the at least one eye of the child,
e) displaying third information on the display screen (5), at or close to the previous location of the second information, and optionally displaying again the second information on the display screen (5), at its previous location,
f) removing the third information from the display screen (5),
g) repeating steps b) to f) a finite number of times,
h) calculating a score in function of the plurality of recorded movements of the at least one eye of the child obtained in the steps d).

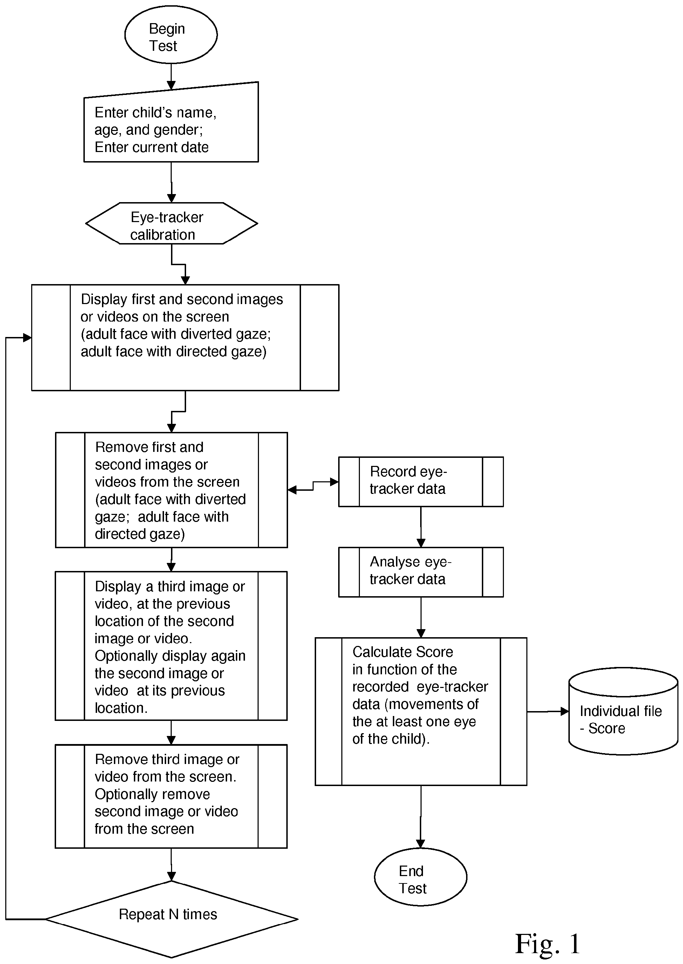

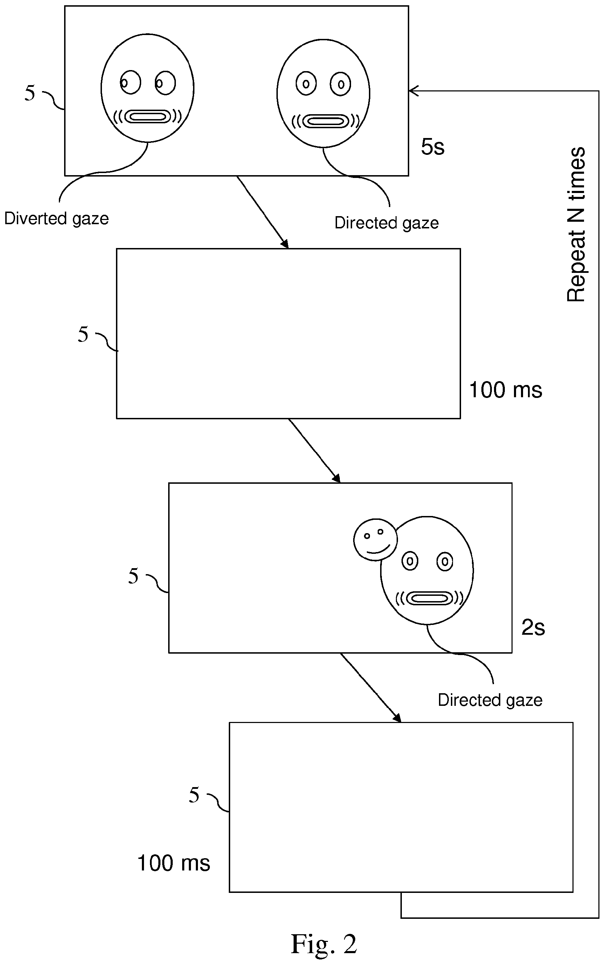

Fig.1 shows a flowchart of an exemplary method according to the invention which will now be described in more detail. Fig.2 shows an example of a series of display screens that are shown to a child during execution of the method of Fig.1.
An exemplary test is a test wherein the attention to eye-region in speech is evaluated. This test is preferably performed when the child is between 10 and 16 months old, preferably when he is between 12 and 14 months old. To this end, the machine is switched ON and the child is instructed to look at the display screen (5) while the eye tracking device is oriented towards his eyes. As shown on Fig.2, two videos of the face of a person uttering (a) word(s) or (a) syllable(s) are then displayed simultaneously on two different parts of the display screen (5) (one video for each face), for example a first video on a left half and a second video on a right half of the display screen (5). These first and second videos constitute in this example what we previously called respectively "the first information" and "the second information". Meanwhile, a loudspeaker (30) plays the sound corresponding to the uttered word(s) or syllable(s). The loudspeaker (30) may for example be a loudspeaker built in into the machine such as the loudspeaker built into a portable computer or into a tablet computer for example.
The eyes of the face displayed on one part of the display screen (5) (for example the right part) are oriented towards the child (directed gaze video) and the eyes of the face displayed on the other part of the display screen (5) (for example the left part) are oriented away from the child (diverted gaze video). In this example, the two videos are played simultaneously for 5 seconds.
Next, the display screen (5) is cleared or the two videos are removed from the display screen (5) for a while, in this example for 100 ms. In either case an optional additional step is to display two other images or videos at the location where respectively the two videos were displayed. One may for example display two small squares or rectangles at respectively these two locations.
While the display screen (5) is cleared or while the two videos are removed from the display screen (5), the controller (15) is reading and recording eye position data from the eye-tracking device (10), thereby obtaining a recorded movement of the at least one eye of the child.
Next, a third information is displayed on the display screen (5), at a location on the display screen (5) which is at least partially there where the second information was previously displayed or close to where the second information was previously displayed. Optionally the second video is again displayed there where it was previously displayed. As part of a third information, a third video or a third image may for example be displayed at least partially in superposition with or close to the previously displayed second video. By "close to the previously displayed second video" one shall understand "closer to the previously displayed second video than to the previously displayed first video".

This third video and the optional second video constitute in this example what we previously called "the third information".
The display screen (5) is then cleared or the third video (or the third image) are removed from the display screen (5) for a while, in this example for 100 ms. Optionally, the second video is also removed from the display screen (5) for the same while, in this example for 100 ms.

As shown on Fig.1 and on Fig.2, the above sequence is repeated a finite number of times, such as 10 times for example.
In parallel with one or several of the above steps, or at a later stage after finalizing the above steps, the plurality of recorded movements of the at least one eye of the child are analysed and a score is calculated in function of said plurality of recorded movements.
The score of the child is then preferably stored in an individual file of the child.

When analysing the recorded movements of the at least one eye of the child, the controller (15) may for example look for fixations by the child on the location where the third video or the third image were previously displayed (anticipatory fixations).
The score may for example be equal to or a function of a number of said specific fixations at the end of the test, or of a ratio of said number of specific fixations with respect to a number of fixations on other parts of the screen, at the end of the test.
Alternatively, the score may for example be equal to or a function of an evolution of a number of said specific fixations over time, within a single test or over a plurality of tests.
Alternatively, the score may for example be equal to or a function of a total duration of said specific fixations at the end of the test, or of a ratio of said total duration with respect to the total duration of fixations on other parts of the screen, at the end of the test.
A fixation corresponds to a situation where it is considered that the eye of the child doesn't move significantly with respect to a target object or location in his visual field, i.e. a phase during which the child continuously looks at said target or location for a minimum duration (all within a given tolerance of course). Most eye-tracking devices include a function of detection of fixations with pre-set parameters which can generally be modified by the user.
The duration of a fixation is therefore the duration of said phase.

Hence, the method of the invention addresses the problem that lack of preferential gaze on directed gaze videos may be caused by factors independent of audio-visual integration, such as, for instance, interest towards the diverted video side.
The said scores may each represent a standardized score of attention to eye-region in speech of the child.

Preferably, the method according to the invention further comprises the step of reading (or inputting) and recording identification data of the child under test such as for example his name, birth date, gender, etc..., before starting the test and the step of linking said data with the score obtained for said child. This allows to easily retrieve individual scores based on the identity of the child.

Preferably, the method according to the invention further comprises the step of inputting the current age of the child before starting the said test and the controller (15) checks the inputted age of child and selects said first and second information in function of the current age of the child.

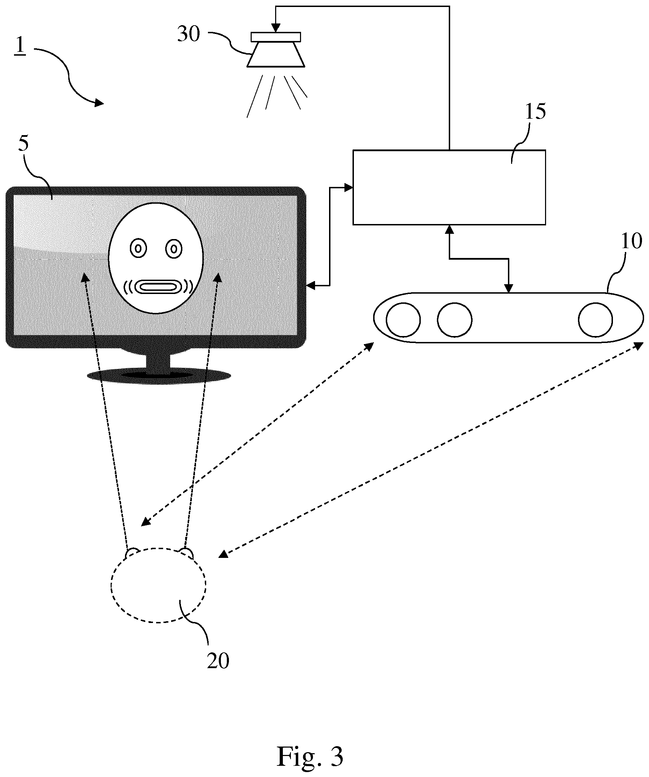

The invention also provides an apparatus for evaluating a risk of neurodevelopmental disorder with a child (20).
An exemplary apparatus (1) is schematically shown on Fig.3. It comprises a display screen (5), an eye-tracking device (10) and a controller (15). The controller (15) is configured for, in the course of a first time period:
a) displaying first information and second information at respectively two distinct locations on the display screen (5),
b) removing the first information and the second information from the display screen (5),
c) while the first information and the second information are removed from the display screen (5), reading and recording eye position data from the eye-tracking device (10), thereby obtaining a recorded movement of the at least one eye of the child,
d) displaying third information on the display screen (5), at or close to the previous location of the second information, and optionally displaying again the second information on the display screen (5), at its previous location,
e) removing the third information from the display screen (5),
f) repeating steps a) to e) a finite number of times,
g) calculating a score in function of the plurality of recorded movements of the at least one eye of the child obtained in the steps c).

Such an apparatus (1) is adapted to perform a method according to the invention as previously described and therefore presents the corresponding advantages compared to conventional apparatus.

Preferably, the apparatus (1) is for evaluating a risk of an Autism Spectrum Disorder (ASD) with a child (20).

Preferably, the first time period is a within a period during which the child (20) has an age comprised between 7 and 20 months, preferably between 9 and 18 months.

Preferably, the first time period is a within a period during which the child (20) has an age comprised between 9 and 11 months, or between 12 and 14 months, or between 16 and 18 months.

Preferably, the apparatus (1) further comprises a loudspeaker (30) and the controller (15) is configured for playing a sound via the loudspeaker (30) while displaying first and/or second and/or third information on the display screen (5).

It will be obvious that other parameters than fixations may be used to establish the score for a child (20) under test, such as a number of saccades, pupil dilation or the amount of time spent looking at the display screen (5) for example.

The present invention has been described in terms of specific embodiments, which are illustrative of the invention and not to be construed as limiting. More generally, it will be appreciated by persons skilled in the art that the present invention is not limited by what has been particularly shown and/or described hereinabove.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

The invention may also be described as follows: a method to evaluate a risk of child having a neurodevelopmental disorder. Firstly, a first and a second information are shown to the child at two different places on a display screen (5). Then the first and second information are removed from the display screen (5) for a while and meanwhile movements of the eye of the child are tracked and recorded. Next, a third information is displayed on the display screen (5), at a location where the second information was previously displayed. This sequence is repeated a number of times. Next, a score for the child is established in function of the movements of his eye(s) as recorded while the first and second information were removed from the display screen (5). This score gives an indication of a risk of neurodevelopmental disorder with the child.

## Claims

1. Method for evaluating a risk of developmental disorder with a child, the method comprising the steps of :
- providing a machine comprising a display screen (5), an eye-tracking device (10), a controller (15) configured for displaying information on the display screen (5) and for reading and recording eye position data from the eye-tracking device (10);
- having the child passing a test in the course of a first time period, the test comprising, in sequence:
a) having the child looking at the display screen (5) and directing the eye-tracking device (10) towards at least one eye of said child,
b) displaying first information and second information at respectively two distinct locations on the display screen (5),
c) removing the first information and the second information from the display screen (5),
d) while the first information and the second information are removed from the display screen (5), reading and recording eye position data from the eye-tracking device (10), thereby obtaining a recorded movement of the at least one eye of the child,
e) displaying third information on the display screen (5), at or close to the previous location of the second information, and optionally displaying again the second information on the display screen (5), at its previous location,
f) removing the third information from the display screen (5),
g) repeating steps b) to f) a finite number of times,
h) calculating a score in function of the plurality of recorded movements of the at least one eye of the child obtained in the steps d).

2. Method according to claim 1, **characterized in that**, based on the recorded movement of the at least one eye of the child during each step d), the controller (15) determines if the child made a fixation on the location on the display screen (5) which was at least partially there where the second information was displayed during respectively each step b), and **in that**, in step h), the score is calculated in function of a number of said fixations or on an evolution of a number of said fixations over time.

3. Method according to claim 1, **characterized in that**, **in that** in step h), the score is calculated in function of a comparison of data from the plurality of recorded movements of the at least one eye of the child obtained in the steps d) with pre-recorded reference data.

4. Method according to any of preceding claims, **characterized in that** the developmental disorder is an Autism Spectrum Disorder.

5. Method according to any of preceding claims, **characterized in that**, in step b), the first and the second information are displayed simultaneously on the display screen (5).

6. Method according to any of preceding claims, **characterized in that** the first time period is a within a period during which the child has an age comprised between 7 and 20 months, preferably between 9 and 18 months.

7. Method according to claim 6, **characterized in that** the first time period is a within a period during which the child has an age comprised between 9 and 11 months, or between 12 and 14 months.

8. Method according to any of preceding claims, **characterized in that** it further comprises the step of recording identification data of the child before starting the said test and the step of linking said data with the said score obtained for said child.

9. Method according to any of preceding claims, **characterized in that** it further comprises the step of inputting the current age of the child before starting the said test and **in that** the controller (15) checks the inputted age of child and selects said first and second information in function of the current age of the child.

10. Apparatus (1) for evaluating a risk of neurodevelopmental disorder with a child, said apparatus (1) comprising a display screen (5), an eye-tracking device (10) and a controller (15), said controller (15) being configured for:
in the course of a first time period:
a) displaying first information and second information at respectively two distinct locations on the display screen (5),
b) removing the first information and the second information from the display screen (5),
c) while the first information and the second information are removed from the display screen (5), reading and recording eye position data from the eye-tracking device (10), thereby obtaining a recorded movement of the at least one eye of the child,
d) displaying third information on the display screen (5), at or close to the previous location of the second information, and optionally displaying again the second information on the display screen (5), at its previous location,
e) removing the third information from the display screen (5),
f) repeating steps a) to e) a finite number of times,
g) calculating a score in function of the plurality of recorded movements of the at least one eye of the child obtained in the steps c).

11. Apparatus (1) according to claim 10, **characterized in that**, based on the recorded movement of the at least one eye of the child during each step c), the controller (15) is configured to determine if the child made a fixation on the location on the display screen (5) which was at least partially there where the second information was displayed during respectively each step a), and **in that**, in step g), the score is calculated in function of a number of said fixations or on an evolution of a number of said fixations over time.

12. Apparatus (1) according to claim 10, **characterized in that** the controller (15) is configured such that, in step g), the score is calculated in function of a comparison of data from the plurality of recorded movements of the at least one eye of the child obtained in the steps c) with pre-recorded reference data.

13. Apparatus (1) according to any of claims 10 to 12, **characterized in that** the developmental disorder is an Autism Spectrum Disorder.

14. apparatus (1) according to any of claims 10 to 13, **characterized in that** the first time period is a within a period during which the child has an age comprised between 7 and 20 months, preferably between 9 and 18 months.

15. Apparatus (1) according to claim 14, **characterized in that** the first time period is a within a period during which the child has an age comprised between 9 and 11 months, or between 12 and 14 months, or between 16 and 18 months.
